# FASCICULE DE BREVET EUROPEEN

(11) **EP 2 352 487 B1**
(45) Date de publication et mention de la délivrance du brevet: **10.05.2017**
(21) Numéro de dépôt: 09774759.6
(22) Date de dépôt: 03.12.2009
(51) Int. Cl.: A61K 8/67, A61K 8/97, A61Q 19/06, A61Q 19/08, A61K 31/01, A61K 31/355, A61K 31/375, A61K 36/15, A61P 17/02

(54) **ASSOCIATION DE LYCOPENE, POLYPHENOL ET VITAMINES POUR LE SOIN DES MATIERES KERATINIQUES**
KOMBINATION VON LYCOPEN, POLYPHENOL UND VITAMINEN ZUR PFLEGE VON KERATINMATERIAL
COMBINATION OF LYCOPENE, POLYPHENOL, AND VITAMINS FOR THE CARE OF KERATIN MATERIAL

(30) Priorité: 03.12.2008 FR 0858235; 16.12.2008 US 122985 P
(43) Date de publication de la demande: 10.08.2011
(73) Titulaire: NUTRICOS Technologies, 92117 Clichy Cedex (FR)
(72) Inventeur: MANISSIER, Patricia, F-92300 Levallois-perret (FR); MONTASTIER, Christiane, F-75016 Paris (FR); PICCIRILLI, Antoine, F-86000 Poitiers (FR)
(74) Mandataire: Nony
(86) Numéro de dépôt international: PCT/IB2009/055489
(87) Numéro de publication internationale: WO 2010/064210

(56) Documents cités:
- EP-A1- 1 090 628
- WO-A1-2006/000226
- WO-A2-2007/112996
- JP-A- 2003 146 899
- US-B1- 6 605 296
- SEGGER D ET AL: "SUPPLEMENTATION WITH EVELLE IMPROVES SKIN SMOOTHNESS AND ELASTICITY IN A DOUBLE-BLIND, PLACEBO-CONTROLLED STUDY WITH 62 WOMEN" JOURNAL OF DERMATOLOGICAL TREATMENT, BASINGSTOKE, GB, vol. 15, no. 4, 1 janvier 2004 (2004-01-01), pages 222-226, XP009085170 ISSN: 0954-6634
- CHOI ET AL: "Cosmeceuticals" SEMINARS IN CUTANEOUS MEDICINE AND SURGERY, W.B. SAUNDERS, PHILADELPHIA, US, vol. 25, no. 3, 1 septembre 2006 (2006-09-01), pages 163-168, XP005703032 ISSN: 1085-5629
- LIU D ET AL: "The scavenging capacity and synergistic effects of lycopene, vitamin E, vitamin C, and beta-carotene mixtures on the DPPH free radical" LEBENSMITTEL WISSENSCHAFT UND TECHNOLOGIE, ACADEMIC PRESS, LONDON, GB, vol. 41, no. 7, 1 septembre 2008 (2008-09-01), pages 1344-1349, XP022650056 ISSN: 0023-6438
- DATABASE WPI Thomson Scientific, London, GB; AN 2006-740227 XP002532094 "ealthy and functional foods exhibiting strong synergistic effect on improving wrinkle and inhibiting wrinkle formation, containing pine tree extract, ascorbic acid, vitamin e and gamma-linolenic acid" & KR 200 622 369 A (LG HOUSEHOLD & HEALTHCARE LTD) 10 mars 2006 (2006-03-10)
- FUCHS J ET AL: "Modulation of uv-light-induced skin inflammation by D-alpha-tocopherol and L-ascorbic acid: A clinical study using solar simulated radiation" FREE RADICAL BIOLOGY AND MEDICINE, ELSEVIER SCIENCE, vol. 25, no. 9, 1 décembre 1998 (1998-12-01), pages 1006-1012, XP002283436 ISSN: 0891-5849
- Anonymous: "Evelle", Internet , 21 November 2007 (2007-11-21), Retrieved from the Internet: URL:http://nutrient.co.uk/evelle.shtml [retrieved on 2015-03-18]
- Anonymous: "The first documented pill against wrinkles - 27.01.2008", Internet , 27 January 2008 (2008-01-27), Retrieved from the Internet: URL:http://www.pharmanord.com/news/2008/th e-first-documented-pill-against-wrinkles-2 7-01-2008 [retrieved on 2015-03-18]

## Description

La présente invention concerne le domaine des compléments nutritionnels destinés au soin de la peau. Elle vise ainsi l'utilisation d'une composition pour administration orale comprenant une association d'actifs destinée à prévenir et/ou traiter les signes cutanés choisis parmi : les rides ou les ridules au niveau des yeux, et les poches ou les cernes sous les yeux.

La peau humaine est constituée de trois compartiments l'épiderme, le derme et l'hypoderme. Ce dernier compartiment est essentiellement constitué d'un type de cellules spécialisées dans l'accumulation et le stockage des graisses, les adipocytes. L'hypoderme est le réservoir énergétique de l'organisme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué par la peau.

Le derme fournit à la peau un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste.

On y trouve aussi des leucocytes, des mastocytes ou encore des macrophages tissulaires. Il est également traversé par des vaisseaux sanguins et des fibres nerveuses. Dans une peau normale, c'est à dire non pathologique ni cicatricielle, le fibroblaste est à l'état quiescent, c'est à dire non prolifératif.

Ce sont les fibres de collagène qui assurent la solidité du derme. Les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.

Les fibres de collagène sont constamment renouvelées mais ce renouvellement diminue avec l'âge ce qui entraîne un amincissement du derme. Cet amincissement du derme est également dû à des causes pathologiques comme par exemple l'hypersécrétion d'hormones corticoïdes, certaines pathologies ou encore des carences vitaminiques (cas de la vitamine C dans le scorbut). Il est également admis que des facteurs extrinsèques comme les rayons ultraviolets, le tabac ou certains traitements (Glucocorticoïdes, vitamine D et dérivés par exemple) ont également un effet sur la peau et sur son taux de collagène.

Divers facteurs entraînent la dégradation du collagène, avec toutes les conséquences que l'on peut envisager sur la structure et/ou la fermeté de la peau et/ou des muqueuses.

Bien que très résistantes, les fibres de collagène sont sensibles à certaines enzymes appelées collagénases. Une dégradation des fibres de collagène entraîne l'apparence de peau molle et ridée que l'être humain, préférant l'apparence d'une peau lisse et tendue, cherche depuis toujours à combattre.

Les collagénases font partie d'une famille d'enzymes appelées métalloprotéinases (MMPs) qui sont elles-mêmes les membres d'une famille d'enzymes protéolytiques (endoprotéases ou endopeptidases) qui possèdent un atome de zinc coordonné à 3 résidus cystéine et une méthionine dans leur site actif et qui dégradent les composants macromoléculaires de la matrice extracellulaire et des membranes basales à pH neutre (collagène, élastine, etc). Très largement répandues dans le monde vivant, ces enzymes sont présentes, mais faiblement exprimées, dans des situations physiologiques normales comme la croissance des organes et le renouvellement des tissus.

Leur surexpression chez l'homme et leur activation est liée à de nombreux processus, parfois pathologiques, qui impliquent la destruction et le remodelage de la matrice. Cela entraîne soit une résorption non contrôlée de la matrice extracellulaire, soit inversement l'installation d'un état de fibrose.

La famille des métalloprotéinases est constituée de plusieurs groupes bien définis basés sur leurs ressemblances en terme de structure et de spécificité de substrat. Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa)), les stromélysines (MMP-3) dont le large spectre d'activité s'adresse aux protéines de la matrice extracellulaire telles que les glycoprotéines (fibronectine, laminine), les protéoglycanes, etc., ou encore les métalloprotéinases membranaires.

En outre, certains protéoglycanes tels que ceux qui appartiennent à la famille des Small Leucine-Rich Proteoglycans (SLRP), constituent une cible intéressante en vue de prévenir les effets négatifs du vieillissement et la détérioration des propriétés mécaniques de la peau. Ces SLRP sont en effet directement impliqués dans la fibrillogénèse et l'hydratation des espaces périfibrillaires. Les SLRP contribuent notamment à accroître la biodisponibilité de certains facteurs de croissance tels que le TGF-β : parmi les SLRP, on peut citer la décorine, le lumican, la fibromoduline, le biglycan. Par ailleurs, certaines observations immunohistochimiques démontrent une diminution de l'accumulation de biglycan dans la peau âgée. De même, la diminution marquée de lumican et de fibromoduline induit une altération de la fibrillogénèse du collagène ainsi qu'une perturbation de l'architecture fibrillaire. Par conséquent, les protéoglycans de la famille des SLRP jouent un rôle fondamental dans l'organisation architecturale des structures de la peau.

Enfin, les SLRPs sont non seulement sensibles à l'action des MMPs, mais également à l'action protéolitique des agrécanases ou ADAMTS (A Disentegrin And Metalloprotease with Thrombospondin type I repeat). Certains membres de cette nouvelle famille de metalloprotéases, en particulier ADAMTS 1 et 4, ont été identifiés au niveau de la peau, et ADAMTS4 est connu pour cliver la décorine.

L'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit.

Par ailleurs à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau papyracée" ou de peau qui tire et l'on constate une accentuation des fines rides et ridules de surface. La peau présente un aspect rugueux à la palpation. Enfin la peau présente une souplesse diminuée.

Enfin, chez le sujet en surpoids et plus particulièrement lors de la prise de poids, les adipocytes ont tendance à augmenter rapidement de volume (stockage de quantités croissantes de lipides). Les lobules graisseux se distendent alors peu à peu pour conduire à la formation de travées conjonctives, parallèles entre elles et perpendiculaires à la surface cutanée. La forte pression exercée par les adipocytes sur le derme provoque rapidement une déformation de la surface de la peau. Au plan cutané, ce phénomène dit de cellulite, se traduit par un aspect capitonné donnant par endroit les signes de « peau d'orange ». Enfin, au plan clinique, la cellulite se traduit par une modification de la texture des tissus sous-cutanés et superficiels, caractérisée en particulier par :
- une peau dans sa globalité plus épaisse,
- une peau plus consistante,
- une peau plus sensible pouvant même en fonction du stade d'avancement de la cellulite être douloureuse à la palpation, et/ou
- des tissus cutanés moins mobiles du fait de la perte d'adhérence et de cohésion des couches profondes de la peau.

Aussi, ce phénomène est plus visible chez les femmes car elles ont une peau plus fine avec des travées conjonctives présentant une structure verticale, qui par contraste chez l'homme, ont une structure oblique et inter croisée.

La cellulite, qui est souvent aggravée par le surpoids et l'obésité, est surtout localisée au niveau du bassin et des membres inférieurs (cellulite en « culotte de cheval » ou «pantalon de zouave »). Ces modifications peuvent aussi conduire à des déformations cicatricielles définitives.

L'hypertrophie du tissu adipeux s'accompagne au niveau dermique d'une mise sous tension des réseaux de fibres, entraînant une altération fonctionnelle des cellules résidentes. En effet, cette hypertension entrave les échanges cellulaires, la circulation veineuse et lymphatique par compression de capillaires, si bien que le phénomène s'auto-entretient. A terme, les fibres dégénèrent et la peau perd ses structures fondamentales.

Au plan biologique, lorsque les fibroblastes sont soumis à une tension tissulaire normale, ils synthétisent activement du collagène, de l'élastine, des glycosaminoglycanes, molécules fondamentales qui contribuent à renforcer les tissus de soutien de la peau. De façon analogue, les adipocytes surchargés de lipides exercent aussi une tension sur le derme, entraînant une surproduction de collagène jusqu'à la fibrose.

En revanche, lors d'une perte de poids et notamment au cours des régimes amincissants, le déstockage rapide des adipocytes entraîne une diminution importante de la tension exercée par l'hypoderme sur les tissus de soutien. Par conséquent, le derme n'étant plus sous tension, le tissu conjonctif perd progressivement de sa cohésion : perte d'attache des fibroblastes au collagène, diminution de la quantité de néocollagène, distension des fibres d'élastine, dépolymérisation des protéoglycanes, .... Dès lors, les fibroblastes en moins grande interaction avec les fibres de la matrice extracellulaire, ne reçoivent plus de leur environnement, les signaux d'activité et de réparation qui commandent la synthèse des macromolécules fondamentales du derme. De plus, les fibroblastes ne recevant plus de signaux de leur environnement fibrillaire sécrètent des métalloprotéases matricielles (MMPs), enzymes entraînant la dégradation des structures fibreuses. Ce ralentissement marqué du métabolisme des fibroblastes, ainsi que la dégradation des fibres par les MMPs, se traduisent par voie de conséquence, par une altération des propriétés viscoélastiques ou biomécaniques de la peau (perte de fermeté, de tonicité, d'élasticité, ...).

On comprend alors à la lecture de ce qui précède l'importance du collagène et des glycosaminoglycanes dans la structure des tissus, particulièrement de la peau et/ou des muqueuses, et l'importance qu'il y a à combattre sa dégradation pour ainsi lutter contre une altération des propriétés viscoélastiques ou biomécaniques de la peau (perte de fermeté, de tonicité, ...) qu'elle soit liée ou non au vieillissement cutané, chronobiologique ou photo-induit.

Il est ici décrit une nouvelle association d'actifs utile pour pallier et/ou prévenir une altération des propriétés biomécaniques de la peau et donc la manifestation de désordres cutanés directement liés à une telle altération comme, par exemple, les désordres associés, l'amincissement du derme et/ou la dégradation des fibres de collagène cette dernière conséquence, entraînant l'apparence de peau molle, flasque et/ou ridée.

La présente description s'intéresse plus particulièrement à la prévention et/ou au traitement par voie orale et/ou parentérale des signes cutanés liés à une altération des propriétés viscoélastiques ou biomécaniques de la peau. Elle vise plus particulièrement à maintenir et/ou restaurer les propriétés biomécaniques de la peau.

Plus précisément, les inventeurs ont découvert que l'administration par voie orale et/ou parentérale d'une association de lycopène, de vitamine C, de vitamine E et d'au moins un composé polyphénol en particulier un composé polyphénol issu d'écorce de pin, présente une activité bénéfique sur les matières kératiniques et en particulier les constituants du derme, et permet de lutter contre une altération des propriétés biomécaniques d'une matière kératinique et/ou agit favorablement notamment sur le maintien et/ou la restauration des propriétés biomécaniques d'une matière kératinique, en particulier d'un tissu conjonctif et plus particulièrement de la peau.

Le lycopène est un pigment naturel que l'on trouve dans les fruits mûrs, particulièrement dans la tomate. Il appartient à la famille des caroténoïdes et sa structure est proche de celle du β-carotène.

Le rôle du lycopène dans la maturation des fruits est connu dans l'art antérieur. Le lycopène est utilisé dans des compositions à activité bronzante pour son rôle sur la synthèse de mélanine (WO 97/47278), dans des compositions destinées au traitement de la chevelure et/ou de l'acné pour son activité sur les 5α-réductases (JP-2940964), comme agent anti-radicalaire (JP-A-8-283136) ou encore est utilisé dans des compositions destinées à traiter, de manière préventive et/ou curative, les signes cutanés du vieillissement (EP 1 090 628).

Les composés polyphénols sont notamment connus pour leur fort pouvoir antioxydant et sont couramment utilisés dans des produits cosmétiques. On a également décrit leur rôle dans la prévention des maladies cardiovasculaires par voie orale. Certains d'entre eux sont également utilisés dans des compositions à usage topique par exemple sous forme estérifiée dans le document FR 2 706 478 de sorte à les rendre liposolubles afin d'améliorer leur stabilité à l'oxydation.

La vitamine C (ou acide ascorbique) est connue pour stimuler la synthèse de collagène, en empêchant, en, tant que co-facteur, l'auto-inactivation des enzymes lysine- et proline-hydroxylases et en augmentant la synthèse des ARNm de procollagènes. L'acide ascorbique (ou vitamine C) est également connu pour stimuler la synthèse de l'élastine de la peau ou pour traiter les rides.

Les composés précités sont déjà proposés en association avec d'autres actifs dans des compositions orales, de type compléments alimentaires par exemple, dédiés notamment à procurer aux sujets ainsi traités un bon état physiologique se traduisant notamment par une meilleure protection à l'égard des maladies cardiaques, rhumatismales, circulatoires (US 6,605,296) ou encore un meilleur aspect de la peau en termes de lissage et de douceur.

Du document WO 02/34210 est connue l'utilisation de l'association d'au moins un caroténoïde et de la vitamine C pour traiter les signes cutanés du vieillissement.

Du document WO 2006/000226 est par ailleurs connue une composition pour l'administration orale utile pour traiter les signes cutanés du vieillissement comprenant de la vitamine E, de la vitamine C, et un extrait de thé blanc, ainsi qu'optionnellement un antioxydant extrait de pépins de raisins, de tomate, de soja et/ou de camomille, un extrait comprenant des glycosaminoglycanes et au moins un métal de transition.

Enfin, Segger *et al.* décrivent une composition orale qui comprend, à titre d'actifs, un mélange de vitamines, de caroténoïdes, de sélénium, de zinc, d'acides aminés, de glycosaminoglycanes, d'un extrait de myrtille et de Pycnogenol^{®}, et la propose notamment pour traiter la rugosité et/ou un défaut d'élasticité de la peau (Journal of Dermatological Treatment (2004), 15, 222-226).

D'une manière générale, la perte d'élasticité cutanée est la conséquence de modifications au niveau du réseau de fibres élastiques dermiques mais également de la diminution des glycosaminoglycanes qui contrôlent la fluidité du liquide interstitiel qui entoure les fibres du réseau dermique. A ce titre, elle n'est pas spécifique à une classe de population et peut ainsi apparaître dès le plus jeune âge.

La présente description est pour sa part, plus particulièrement concernée par la protection, voire l'amélioration de la tonicité, fermeté, souplesse et/ou densité de la peau.

Les déficiences en tonicité, fermeté, souplesse et/ou densité de la peau sont la conséquence le plus souvent d'un vieillissement cutané mais également d'un état de fatigue. Ainsi, ces déficiences peuvent être directement à l'origine de l'apparition de signes de vieillissement cutané tels que les rides et/ou ridules qui se manifestent typiquement à partir de 30 ans pour les rides, mais aussi de signes cutanés non directement liés à l'âge mais néanmoins plus particulièrement rencontrés chez l'adulte tels que les poches et les cernes.

En terme de mécanisme biologique, les rides résultent d'une perte musculaire ou de tensions musculaires répétées, d'une perte des tissus graisseux, des forces gravitationnelles persistantes et de la perte des os et du cartilage au niveau du visage. L'aplatissement de la jonction entre le derme et l'épiderme ainsi que la diminution du collagène IV sont considérés comme des facteurs influençant la formation des rides.

D'un point de vue histologique, l'épiderme apparaît atrophié, manifestation distincte de la ptose des tissus cutanés observée lors d'une perte d'élasticité cutanée.

De manière inattendue, les inventeurs ont donc constaté qu'il était possible de traiter la manifestation de ces signes cutanés, à savoir notamment rides, ridules, poches et cernes en stimulant efficacement les propriétés biomécaniques de la peau *via* l'administration d'une composition bien spécifique.

On connaît par ailleurs des traitements topiques permettant de lutter contre les signes cutanés notamment liés au vieillissement. Toutefois, les actifs topiques préconisés n'agissent pas toujours du fait de leur faible pénétration cutanée, au niveau dermique. En outre, les produits topiques agissent par définition localement sur les zones à traiter, zones sur lesquelles ils peuvent être inégalement répartis, et nécessitent des applications soignées et répétées. Ils peuvent être dans certains cas à l'origine de réactions secondaires cutanées, voire d'inconfort.

Par opposition, la voie orale présente l'avantage d'agir de façon globale sur l'ensemble de la peau et ce dans ses couches profondes (derme, hypoderme), suivant un mode d'administration rapide et peu contraignant. En effet, les métabolites et autres nutriments actifs sont en particulier distribués au sein de la matrice dermique par le biais de la circulation sanguine. La voie orale ou l'administration par patch présentent également l'avantage d'un mode d'administration rapide et peu contraignant.

L'association considérée dans cette description permet encore plus particulièrement de maintenir et/ou de restaurer les propriétés de tonicité, de fermeté, de souplesse et/ou de densité de la peau.

L'association selon l'invention concerne plus particulièrement une association de lycopène, de vitamine C, de vitamine E et d'au moins un composé polyphénol issu d'écorce de pin à titre d'unique principe actif.

L'invention a ainsi pour objet l'utilisation cosmétique par voie orale d'une association de lycopène, de vitamine C, de vitamine E et d'au moins un composé polyphénol issu d'écorce de pin à titre d'unique principe actif, destinée à prévenir et/ou traiter les signes cutanés choisis parmi : les rides ou les ridules au niveau des yeux, et les poches ou les cernes sous les yeux.

L'invention a en particulier pour objet l'utilisation d'une telle association pour prévenir et/ou traiter des signes cutanés tels que les rides et les ridules au niveau des yeux et/ou pour prévenir et/ou traiter les signes cutanés de fatigue tels que les poches péri-oculaires, les cernes péri-oculaires et la peau terne et sans éclat, de préférence pour prévenir et/ou traiter les poches péri-oculaires.

Selon un mode de réalisation préféré, l'association considérée selon l'invention permet de prévenir et/ou traiter les signes cutanés de fatigue tels que les poches péri-oculaires, les cernes péri-oculaires et la peau terne et sans éclat.

L'association d'actifs ici décrite permet également d'améliorer la qualité des ongles.

Cette association s'avère tout particulièrement intéressante en termes d'effets bénéfiques sur le maintien et/ou la restauration de la fermeté cutanée chez les femmes ménopausées. Ont ainsi été démontrés un accroissement de l'activité cellulaire du derme, une amélioration de la qualité de la matrice extracellulaire du derme et une meilleure hydratation du gel matriciel de la matrice extracellulaire, consécutivement à l'administration orale d'un complément oral contenant cette association.

La présente description enseigne par ailleurs l'utilisation d'une association de lycopène, de vitamine C, de vitamine E et d'au moins un composé polyphénol issu d'écorce de pin, à titre d'actif pour la préparation d'une composition pour administration orale et/ou parentérale destinée à favoriser la cicatrisation.

En particulier, les compositions décrites sont particulièrement utiles pour maintenir et/ou restaurer les propriétés de tonicité, de fermeté, de souplesse, et/ou de densité de la peau.

Il est par ailleurs ici décrit l'utilisation cosmétique par voie orale d'une association de lycopène, de vitamine C, de vitamine E et d'au moins un composé polyphénol issu d'écorce de pin à titre d'actif destiné à améliorer la qualité des ongles.

Il est aussi décrit une composition pour l'administration orale et/ou parentérale comprenant à titre d'unique principe actif l'association de lycopène, de vitamine C, de vitamine E et d'au moins un composé polyphénol issu d'écorce de pin.

Il est aussi décrit une composition pour administration orale et/ou parentérale comprenant l'association de lycopène, de vitamine C, de vitamine E et d'au moins un composé polyphénol issu d'écorce de pin, dans laquelle la valeur du rapport entre la teneur en poids en composé polyphénol par rapport à la somme des teneurs en poids en lycopène, vitamine C et vitamine E est comprise entre 0,05 et 1,2, en particulier entre 0,2 et 1, et en particulier entre 0,3 et 0,7.

Avantageusement, une telle composition se présente en outre sous forme de capsules molles, de gélules banderolées, de gels, d'émulsions sèches ou liquides, de comprimés, de poudres à diluer ou d'ampoules buvables, ou d'aliments fonctionnels tels que par exemple yaourts, boissons....

Les compositions décrites sont particulièrement utiles pour maintenir et/ou restaurer les propriétés de tonicité, de fermeté, de souplesse et/ou de densité de la peau.

En particulier, l'association de lycopène, de vitamine C, de vitamine E et d'au moins un composé polyphénol issu d'écorce de pin est particulièrement utile pour la préparation d'une composition pour l'administration orale et/ou parentérale destinée à prévenir et/ou traiter les signes cutanés liés à une altération des propriétés précitées.

Par « propriétés viscoélastiques ou biomécaniques de la peau » dans le cadre de la présente invention, on entend les propriétés d'extensibilité, de tonicité, de fermeté et/ou de souplesse de la peau.

Par « signes cutanés », on entend toutes modifications de l'aspect extérieur de la peau, comme par exemple les rides, notamment au niveau des yeux et des commissures des lèvres, les ridules, la peau flétrie, la peau molle, la peau flasque, la peau amincie, la peau terne et sans éclat, les poches sous les yeux, les sillons cutanés, le manque d'élasticité et/ou de tonus de la peau, mais également toutes modifications internes de la peau qui ne se traduisent pas systématiquement par un aspect extérieur modifié, comme par exemple toutes dégradations internes de la peau, particulièrement des fibres de collagène, consécutives à une exposition aux rayonnements ultraviolets.

Par « signes cutanés de fatigue » on entend toutes les modifications de l'aspect extérieur de la peau induites et/ou accentuées par une grande fatigue, un manque de sommeil et/ou une vie stressante, comme par exemple les poches péri-oculaires, les cernes péri-oculaires ou encore la peau terne et sans éclat.

Par « désordre cutané induit par les régimes amaigrissants et/ou amincissants », on entend toutes les modifications de l'aspect extérieur de la peau comme par exemple l'aspect peau flasque qui peut être plus ou moins marqué consécutivement à une perte de poids.

Par « désordre cutané induit par la cellulite », on entend toutes les modifications de l'aspect extérieur de la peau comme par exemple les capitons ou « peau d'orange » qui peuvent être plus ou moins localisés dans les zones de surcharge pondérale telles que les cuisses, les bras ou l'abdomen.

Il est entendu dans le cadre de la présente invention que « l'utilisation cosmétique par voie orale » recouvre l'utilisation de produits administrés par voie orale, ces produits, par exemple sous forme de complément alimentaire ou d'aliment fonctionnel comme exposé ci-après pour le cas de la voie orale, produisant un effet au niveau de la peau sur le plan esthétique et du confort, ou encore à visée beauté, par exemple en vue de la protéger, de la maintenir en bon état, d'en modifier l'aspect, et notamment de l'embellir.

Dans le cadre de la présente description, la voie parentérale s'entend de l'injection intramusculaire, de l'injection intraveineuse ou encore de l'administration par patch à visée systémique. Autrement dit, cette définition entend couvrir tous les autres modes d'administration autres que la voie orale (ou digestive) pour autant que les actifs passent par la circulation sanguine.

L'administration par patch à visée systémique est préférée à titre d'administration parentérale. Le patch à effet exclusivement local est exclu de la présente invention.

Par « dispositif transdermique », « patch » ou « système de délivrance transdermique » au sens de l'invention, on entend tout système permettant une libération active ou passive de la substance active par transdermie, c'est-à-dire permettant son transfert à travers la peau jusqu'à la circulation générale systémique.

### LYCOPENE

Le lycopène utilisé selon l'invention peut être d'origine naturelle ou synthétique. Par origine naturelle, on entend le lycopène, à l'état pur ou en solution quelle qu'en soit sa concentration dans ladite solution, obtenu à partir d'un élément naturel comme par exemple un extrait végétal, particulièrement la tomate. Par origine synthétique, on entend le lycopène, à l'état pur ou en solution quelle qu'en soit sa concentration dans ladite solution, obtenu par synthèse chimique.

Lorsque le lycopène est d'origine naturelle, il peut être obtenu à partir de matériel végétal issu de plante entière cultivée *in vivo* ou issu de culture *in vitro.*

Par cultivée *in vivo* on entend toute culture de type classique c'est à dire en sol à l'air libre ou en serre, ou encore hors sol.

Par culture *in vitro,* on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Préférentiellement selon l'invention, on utilise un végétal issu de culture *in vivo.* Très préférentiellement selon l'invention, on utilise un extrait de tomate riche en lycopène.

Le lycopène est également présent dans le melon, la goyave et le pamplemousse.

Toute méthode d'extraction connue de l'homme du métier peut être utilisée pour préparer le lycopène utilisé selon l'invention.

Le lycopène peut être en suspension aqueuse. Pour cela, on peut utiliser des formes hydrodispersibles, à froid ou à chaud, telles que commercialisées par la Société Lycored sous les dénominations Lyc-o-Mato CWD^{®}.

A titre d'exemple, selon l'invention on peut utiliser un extrait de tomate riche en lycopène, préparé par la société Lycored, commercialisé sous la dénomination LycOMato^{®} constitué d'un extrait d'oléorésine contenant par exemple de 6 à 10 % de lycopène pur.

Tout autre ingrédient plus complexe à base de lycopène peut également être utilisé pour la mise en oeuvre de l'invention.

Ainsi, on entend au titre d'ingrédient plus complexe par exemple une composition primaire comprenant le lycopène et une protéine de petit lait. Cette composition primaire est notamment décrite dans le document WO 01/91588. Cette composition primaire porte également le nom de lactolycopène. C'est cet ingrédient qui est utilisé dans le complément alimentaire de l'exemple 1. Il présente l'intérêt d'augmenter la biodisponibilité du lycopène et/ou d'être aisément formulable dans les compléments alimentaires (formes sachet, gélule, comprimé, dragée, capsule molle, ...)

La quantité d'extrait utilisable selon l'invention est bien entendu fonction de l'effet recherché et peut donc varier dans une large mesure.

Pour donner un ordre de grandeur, on peut utiliser le lycopène à l'état pur en une quantité représentant de 0,0001 à 50 % en poids, de préférence de 0,001 à 10 % en poids, préférentiellement en une quantité représentant de 0,05 à 0,2 % en poids par rapport au poids total de la composition.

Par exemple, lorsque la composition est plus particulièrement destinée à prévenir et/ou traiter les signes cutanés de fatigue tels que les poches péri-oculaires, les cernes péri-oculaires et la peau terne et sans éclat, on peut utiliser le lycopène à l'état pur en une quantité représentant de 0,001 à 1 % en poids, notamment de 0,005 à 0,1 % en poids par rapport au poids total de la composition.

Bien entendu l'homme du métier, s'il utilise le lycopène sous la forme d'une solution, un extrait végétal par exemple, sait ajuster la quantité de solution qu'il utilise dans sa composition afin que la quantité finale de lycopène dans la composition soit en accord avec les quantités utilisables précédemment définies.

### VITAMINE C

Selon l'invention, la vitamine C ou l'acide ascorbique et/ou ses analogues peuvent être utilisés seuls ou en mélanges de toute nature et toute proportion et peuvent être d'origine naturelle ou synthétique.

L'acide ascorbique est généralement sous forme L, car c'est particulièrement sous cette forme qu'il est retrouvé dans le monde végétal.

La quantité de vitamine C utilisable selon l'invention est bien entendu fonction de l'effet recherché et peut donc varier dans une large mesure.

Pour donner un ordre de grandeur, dans la composition de l'invention la vitamine C à l'état pur peut être présente en une teneur allant de 0,0001 à 50 % en poids, de préférence de 0,1 à 10 % en poids, et préférentiellement en une teneur allant de 3 % à 6 % en poids par rapport au poids total de la composition.

Par exemple, lorsque la composition est destinées à prévenir et/ou traiter les signes cutanés de fatigue tels que les poches péri-oculaires, les cernes péri-oculaires et la peau terne et sans éclat, la vitamine C à l'état pur peut être présente en une teneur allant de 0,001 à 5 % en poids, notamment de 0,05 à 1 % en poids par rapport au poids total de la composition.

Bien entendu, si la vitamine C est présente sous la forme d'une solution, par exemple un extrait de plante, l'homme du métier saura ajuster la quantité de cette solution dans la composition selon l'invention, de façon à obtenir les gammes de concentrations en vitamine C ci-dessus décrites.

### VITAMINE E

La vitamine E peut être présente dans la composition pour administration orale et/ou parentérale dans une teneur allant de 0,0001 à 50 % en poids, de préférence de 0,1 à 10 % en poids, de façon encore plus préférée de 0,5 à 2 % en poids par rapport au poids total de la composition.

Par exemple, lorsque la composition est destinée à prévenir et/ou traiter les signes cutanés de fatigue tels que les poches péri-oculaires, les cernes péri-oculaires et la peau terne et sans éclat, la vitamine E peut être présente en une teneur allant de 0,001 à 5 % en poids notamment de 0,005 à 1 % en poids par rapport au poids total de la composition.

### COMPOSE POLYPHENOL

Les composés polyphénols regroupent une grande famille de composés très largement répandus dans le règne végétal. On les trouve ainsi notamment dans les plantes, depuis les racines jusqu'aux fruits. Parmi les classes de polyphénols, on peut notamment citer les flavonoïdes, les proantocyanidines, les lignanes, les lignines, les stilbènes, les coumarines. Ainsi, le composé polyphénol mis en oeuvre dans le cadre de la présente invention peut se présenter sous toutes ces formes précitées.

Les composés polyphénols peuvent notamment dériver d'extraits végétaux choisis parmi les extraits de thé vert, de raisin tels que le *Vitis Vinifera,* de pin et notamment d'écorce de pin, de pomme, de myrtille, de houblon, de goyave, de cacao, de bois tels que le châtaignier, le chêne, le marronnier d'Inde, le noisetier.

Le terme « composé polyphénol » dans le cadre de la présente invention s'étend donc également à l'extrait végétal lui-même, riche en ces composés polyphénols.

Les flavonoïdes représentent le principal groupe de polyphénols.

Les polyphénols catéchiques constituent, quant à eux, un sous groupe des flavonoïdes, qui comprennent également les flavanones, les flavones et anthocyanines, et les flavonols.

Le composé polyphénol est précisément un polyphénol catéchique tel que défini ci-après.

Le sous groupe des polyphénols catéchiques comprend un ensemble de composés classiquement isolés de plantes telles que le cacao, le thé, la vigne et ses dérivés, le pin (*Pinus maritima*)*,* le cachou, certains fruits et présentant un degré de polymérisation variable.

L'unité de base, aussi appelée catéchine ou catéchol est le penta hydroxy-3,5,7,3',4' dihydro-2,3 phényl-2 chromène, qui peut être sous forme cis ou trans; l'épicatéchine est son isomère, et peut également être présent sous forme cis ou trans.

Les polyphénols catéchiques englobent aussi bien les divers isomères des unités de base (monomères), que des oligomères (ou proanthocyanidols) ou des polymères (tannins).

Plus particulièrement, les polyphénols catéchiques utiles selon l'invention sont choisis dans le groupe comprenant : catéchine, épicatéchine, gallocatéchine, épigallocatéchine et leurs sels, leurs esters et/ou leurs dérivés, sous forme monomères ou oligomères.

Lorsque des oligomères sont utilisés, ils comportent avantageusement de 2 à 14 unités de base, notamment de 2 à 10.

De préférence leur degré de polymérisation est inférieur ou égal à 5.

On utilise en particulier des composés généralement appelés proanthocyanidoles ou procyanidols, encore appelés précurseurs d'anthocyanines ou oligomères procyanidoliques (OPC). Ces oligomères seront dégradés, pour une partie, après absorption par voie orale et/ou parentérale pour libérer les monomères.

Ces polyphénols peuvent être conjugués avec des sucres comme par exemple glucose, galactose, rhamnose, acide galacturonique.

Par polyphénols catéchiques on entend notamment dans le présent texte des mélanges en toutes proportions de monomères et des différents oligomères comportant de 2 à 14 unités, tels que définis précédemment.

Parmi les dimères largement répandus de la famille des procyanidols ou oligomères procyanidoliques et dont la mise en oeuvre est particulièrement avantageuse dans le cadre de la présente invention, on peut citer la procyanidine B1, la procyanidine B2, la procyanidine B3 ou encore la procyanidine B6 ou B7.

Les procyanidines B1, B2, B3 sont présentes dans des extraits végétaux d'écorce de pin, de cacao, de pomme, de myrtille, de marronnier d'Inde, de houblon, de goyave et de noisetier. Ainsi, la composition selon la présente invention comprend avantageusement un de ces extraits végétaux.

Le composé polyphénol présent dans la composition objet de la présente invention est issu d'écorce de pin.

Un tel composé polyphénol issu d'écorce de pin présente avantageusement une teneur en trimères phénoliques pouvant aller de 5 à 25 % en poids, de préférence de 10 à 20 % en poids par rapport au poids total du composé polyphénol. De même, il présente avantageusement une teneur en dimères polyphénoliques d'au moins 5 % en poids ou pouvant aller de 5 à 25 % en poids, de préférence d'au moins 10 % en poids ou pouvant aller de 10 à 20 % en poids par rapport au poids total du composé polyphénol.

Le composé polyphénol issu d'écorce de pin contient aussi avantageusement de 2 à 15 % en poids, par exemple de 5 à 10 % en poids, d'acides phénoliques de type acide ferrulique, acide p-coumarique, acide caféique et acide protocatéchique, par rapport au poids total du composé polyphénol.

Ainsi, le composé polyphénol, particulièrement avantageux pour la mise en oeuvre de l'invention, peut présenter les caractéristiques suivantes :

| **Analyse/critère** | **Spécification** |
|---|---|
| Perte à la dessiccation | ≤ 5,0 % |
| Cendres sulfuriques | ≤ 0,4 % |
| Insolubles dans l'eau (solution à 1 %, T = 37 °C) | ≤ 5,0 % |
| Insolubles dans THF (solution à 1 %, T = 20 °C | ≤ 1,0 % |
| pH (solution aqueuse à 4 %, T = 20 °C) | 2,5 - 4,5 |
| Trimères polyphénoliques | 10-20 % |
| Dimères polyphénoliques | 10-20 % |
| Taxifoliol + taxifoliol glucoside | > 3 % |
| Teneurs en acides phénoliques ⁽¹⁾ | 2-15 % |

| | |
|---|---|
| ⁽¹⁾ acides ferrulique + p-coumarique + protocatéchique + caféique. | |

Aussi, selon une variante de réalisation de l'invention, le composé polyphénol issu d'écorce de pin provient d'un extrait de pin maritime. Un tel extrait de pin maritime est notamment décrit dans l'article « A review of the French Maritime Pine Bark Extract (PYCNOGENOL®), a herbal médication with a diverse clinical pharmacology », P. ROHDEWALD, Internationl Journal of Clinical Pharmacology and Therapeutics, Vol. 40-No 4/2002(158-168),

Une composition ici décrite comprend de préférence le composé polyphénol dans une teneur allant de 0,0001 à 50 % en poids et de préférence de 0,001 à 10 % en poids, de façon encore plus préférée de 0,5 à 2 % en poids par rapport au poids total de la composition.

Par exemple, lorsque la composition est destinée à prévenir et/ou traiter les signes cutanés de fatigue tels que les poches péri-oculaires, les cernes péri-oculaires et la peau terne et sans éclat, le composé polyphénol peut être présent en une teneur allant de 0,001 à 5 % en poids, notamment de 0,1 à 1 % en poids par rapport au poids total de la composition.

Comme l'illustrent les exemples qui suivent, les inventeurs ont démontré qu'une composition contenant du lycopène, de la vitamine C, de la vitamine E et un composé polyphénol issu d'écorce de pin dans le rapport de teneur requis, pouvait agir favorablement sur la matrice dermique, par le biais d'un accroissement de l'activité cellulaire du derme, d'une amélioration de la qualité de la matrice extracellulaire du derme et une meilleure hydratation du gel matriciel de la matrice extracellulaire.

Une composition ici décrite est ainsi destinée à lutter contre les signes cutanés du vieillissement et tout particulièrement destinée à maintenir et/ou à restaurer les propriétés biomécaniques de la peau.

Ainsi, comme précisé précédemment, une composition ici décrite est particulièrement utile pour maintenir et/ou restaurer les propriétés de tonicité, de fermeté, de souplesse et/ou de densité de la peau.

Par conséquent, la dite composition est particulièrement destinée à la prévention et/ou au traitement cosmétique des désordres cutanés induits par le chronovieillissement, en particulier des peaux matures des femmes pré- ou post-ménopausées, mais encore à la prévention et/ou au traitement cosmétique des désordres induits par le photovieillissement.

La dite composition est donc également adaptée à la prévention et/ou au traitement des désordres cutanés induits par la ménopause.

Ainsi, il est aussi décrit l'utilisation cosmétique d'une telle composition pour la prévention et/ou le traitement des désordres cutanés induits par la ménopause.

La dite composition est aussi particulièrement adaptée à la prévention et/ou au traitement cosmétique des désordres cutanés induits par la perte de poids telle qu'observée au cours des régimes amincissantes et/ou amaigrissants, tels que relâchement des tissus de soutien, perte de tonicité et de souplesse cutanée, et visibilité accrue des capitons.

Il est également décrit l'utilisation cosmétique d'une composition conforme à la présente invention à titre de composition destinée à lutter contre le manque de souplesse et/ou de tonus de la peau.

Il est enfin décrit l'utilisation cosmétique d'une composition conforme à l'invention à titre de composition destinée à prévenir et/ou traiter les aspects visuels liés à la cellulite, tels que capitons et « peau d'orange ».

Ainsi qu'illustré en exemple 1, le gène de la vitamine est significativement augmenté, de sorte qu'il apparaît que l'association visée par la présente invention présente une action favorable sur la cicatrisation.

Il est enfin décrit l'utilisation cosmétique d'une telle composition à titre de composition destinée à prévenir et/ou traiter des signes cutanés tels que les rides et les ridules et/ou à prévenir et/ou traiter les signes cutanés de fatigue tels que les poches péri-oculaires, les cernes péri-oculaires et la peau terne et sans éclat.

Selon un mode de réalisation privilégié, le lycopène est présent dans cette composition dans une teneur allant de 0,05 à 0,2 % en poids, la vitamine C est présente dans une teneur allant de 3 à 6 % en poids, la vitamine E est présente dans une teneur allant de 0,5 à 2 % en poids, et le composé polyphénol est présent dans une teneur allant de 0,5 à 2 % en poids, par rapport au poids total de la composition.

Lorsque la composition est plus particulièrement destinée à prévenir et/ou traiter les signes cutanés de fatigue tels que les poches péri-oculaires, les cernes péri-oculaires et la peau terne et sans éclat, le lycopène est présent dans cette composition dans une teneur allant de 0,005 à 0,1% en poids, la vitamine C est présente dans une teneur allant de 0,05 à 1 % en poids, la vitamine E est présente dans une teneur allant de 0,005 à 1 % en poids, et le composé polyphénol est présent dans une teneur allant de 0,1 à 1 % en poids, par rapport au poids total de la composition.

Selon encore un autre mode de réalisation privilégié, la valeur du rapport entre la teneur en poids en composé polyphénol par rapport à la somme des teneurs en poids en lycopène, vitamine C et vitamine E est comprise entre 0,2 et 1 et de façon encore plus préférée entre 0,3 et 0,7.

Lorsque la composition est plus particulièrement destinée à prévenir et/ou traiter les signes cutanés de fatigue tels que les poches péri-oculaires, les cernes péri-oculaires et la peau terne et sans éclat, la valeur du rapport entre la teneur en poids en composé polyphénol par rapport à la somme des teneurs en poids en lycopène, vitamine C et vitamine E est comprise entre 0,3 et 0,7 et de façon encore plus préférée entre 0,4 et 0,6.

### COMPOSITION

Une association selon l'invention est une association de lycopène, de vitamine C, de vitamine E et d'au moins un composé polyphénol issu d'écorce de pin à titre d'unique principe actif.

Une association selon l'invention est administrée par voie orale. Les compositions correspondantes peuvent se présenter sous toutes les formes galéniques normalement utilisées selon le mode d'administration concerné.

Il peut s'agir d'une composition de type complément alimentaire ou d'un aliment fonctionnel, ou bien d'une composition pharmaceutique.

Une composition conforme à l'invention peut notamment se présenter sous forme de capsules molles, de gélules banderolées, de gels, d'émulsions sèches ou liquides, de comprimés, de poudres à diluer ou d'ampoules buvables ou toute autre forme connue de l'homme du métier. La composition peut éventuellement contenir des excipients de formulation appropriée tels que colorant, édulcorant, aromatisant, agent de charge, liant, conservateur, etc.

Selon un autre mode de réalisation privilégié de l'invention, l'association de lycopène, de vitamine C, de vitamine E et de composé polyphénol extrait de pin, à titre d'unique principe actif, peut aussi être incorporée dans des matrices alimentaires en vu de produire des aliments fonctionnels tels que des barres alimentaires, des aliments enrichis tels que des huiles, des beurres, des margarines, des poudres compactées, des fibres ou encore sous le forme d'émulsion dans des boissons.

Ainsi, la dite composition peut être un aliment fonctionnel.

A titre indicatif, les doses de lycopène apportées pour atteindre les buts de l'invention seront adaptées en fonction de l'effet recherché. Par exemple, on pourra prévoir une dose allant de 1 à 22 mg, par exemple allant de 2 à 11 mg/jour, voire de 4 à 7 mg/jour.

De même, les doses de composés polyphénols sous forme de monomère ou d'oligomère apportées pour atteindre les buts de l'invention peuvent varier de 6 à 75 mg, par exemple de 13 à 75 mg, notamment de 20 à 75 mg/jour, et plus particulièrement de 30 à 50 mg/jour.

De plus, les doses de vitamine C apportées pour atteindre les buts de l'invention peuvent varier de 10 à 105 mg, par exemple de 20 à 105 mg, notamment de 33 à 105 mg/jour, avantageusement de 50 à 70 mg/jour.

Enfin, les doses de vitamine E apportées pour atteindre les buts de l'invention peuvent varier de 1 à 17 mg, par exemple de 3 à 17 mg, notamment de 6 à 17 mg/jour, avantageusement de 9 à 11 mg/jour.

Lorsque l'association selon l'invention est plus particulièrement destinée à prévenir et/ou traiter les signes cutanés de fatigue tels que les poches péri-oculaires, les cernes péri-oculaires et la peau terne et sans éclat, de préférence à prévenir et/ou traiter les poches péri-oculaires, la dose journalière en lycopène peut être comprise entre 2 et 11 mg et/ou la dose journalière en vitamine C peut être comprise entre 20 et 70 mg et/ou la dose journalière en vitamine E peut être comprise entre 3 et 12 mg et/ou la dose journalière en composé polyphénol peut être comprise entre 13 et 50 mg.

Il est ici décrit une composition pour administration orale comprenant une association de lycopène, de vitamine C, de vitamine E et d'au moins un composé polyphénol issu d'écorce de pin, dans laquelle la valeur du rapport entre la teneur en poids en composé polyphénol par rapport à la somme des teneurs en poids en lycopène, vitamine C et vitamine E est compris entre 0,3 et 0,7, caractérisée en ce qu'elle se présente sous forme de capsules molles, de gélules banderolées, de gels, d'émulsions sèches ou liquides, de comprimés, de poudres à diluer ou d'ampoules buvables, ou d'aliments fonctionnels tels que par exemple yaourts, boissons, ...

Au sens de la présente description, une composition cosmétique désigne une composition apte à produire un effet au niveau de la peau sur le plan esthétique et du confort, ou encore à visée beauté, par exemple en vue de la protéger, de la maintenir en bon état, d'en modifier l'aspect, et notamment de l'embellir. Elle peut se présenter sous la forme d'un produit nutritionnel.

La présente invention est illustrée par les exemples qui suivent.

### Exemple 1 : étude clinique exploratoire relative à la quantification des ARNm de référence induits par la prise du complément alimentaire A

### Formule du complément alimentaires A

- Forme galénique : comprimés pelliculés
- Posologie : 2 comprimés/jour
- Formule Complément Alimentaire A

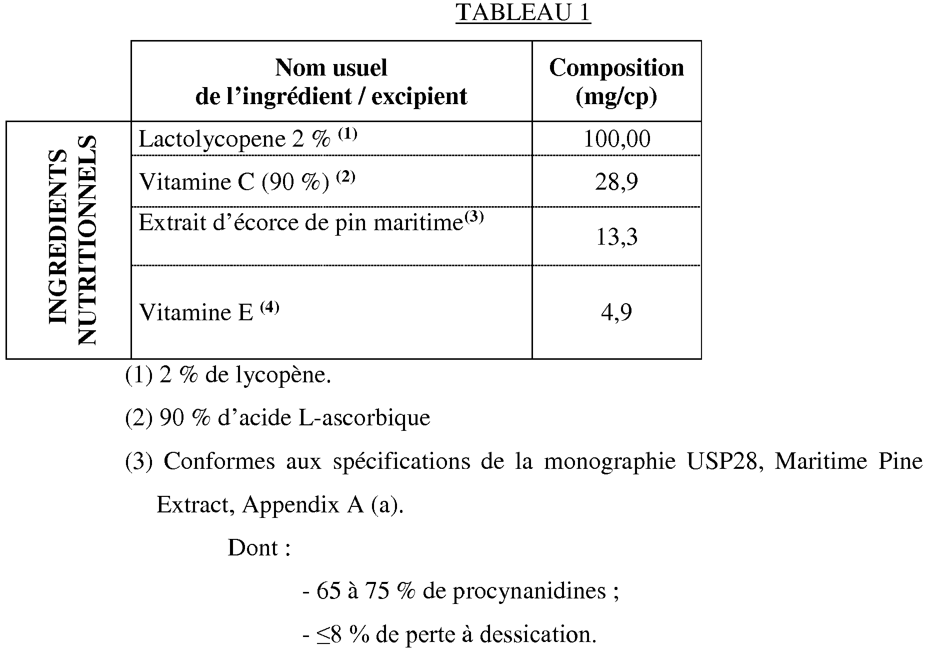

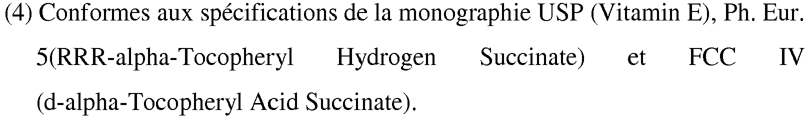

Le complément alimentaire A est préférentiellement destiné aux femmes ménopausées qui sont confrontées à une perte de fermeté cutanée.

### Objectif de l'essai clinique

Evaluer l'effet du complément alimentaire A contre placebo, sur des biomarqueurs cutanés susceptibles d'être associés à un changement des propriétés biomécaniques de la peau après 2 mois de prise du complément alimentaire A.

### Méthodologie

- Etude de 2 mois sur 18 femmes ménopausées depuis plus de deux ans (9 dans le groupe prenant le complément alimentaire A / 9 dans le groupe placebo), âgées de plus de 50 ans, ne suivant pas un traitement hormonal substitutif, et présentant un manque de fermeté cutanée au niveau de la face interne du bras.
- Réalisation de biopsies de 3 mm sur le bras à T0 et T2 mois pour extraction des ARNm totaux.
- Quantification d'ARNms spécifiques codant pour des protéines susceptibles d'être associées à un changement des propriétés biomécaniques de la peau.

### Préparation des ARNm en vue de la RT-PCR

Les deux biopsies ont été poolées, broyées dans de l'azote liquide (Mikro Dismenbrator S, B. Braun Biotech International), puis l'ARN total a été extrait et purifié par ultracentrifugation sur gradient de chlorure de césium. La quantité d'ARN purifié a été évaluée en mesurant la DO (densité optique) à 260 nm (Nanodrop) et la qualité de l'ARN extrait a été validée par le calcul du ratio DO260/DO280. L'intégrité des ARN extraits a également été vérifiée en utilisant le BioAnalyzer de chez Agilent. Des solutions stocks d'ARN ont été préparées afin d'obtenir une concentration proche de 1,25 ng/µl.

### Evaluation de la quantité des biomarqueurs cutanés

La concentration en ARN a été normalisée par rapport à la quantité d'ARN ribosomal 28S. La technique de RT-PCR a été rendue quantitative en ajoutant dans chaque tube de réaction un standard interne de concentration connue, et constitué par une ARN synthétique, qui sera co-transcrit et co-amplifié en même temps que l'ARN recherché. Les échantillons correspondant au T0 et au T2 de chaque volontaire ont été analysés dans la même série, et ont fait l'objet d'un dépôt et d'une migration sur le même gel de polyacrylamide. L'analyse statistique a été réalisée au moyen d'un test de Student unilatéral pour échantillon apparié, en comparant dans chaque groupe les valeurs à T0 et à T2. Un ratio T2/T0 = 1,00 signifie que les deux valeurs sont comparables. Un ratio T2/T0 > 1,00 est le reflet d'une augmentation du transcript étudié. A l'inverse, un ratio T2/T0 < 1,00 est le reflet d'une diminution de l'expression de l'ARNm étudié.

### Résultats / Quantification d'ARNm cibles

**Tableau II : expression des biomarqueurs cibles après prise du complément alimentaire A (versus placebo)**

| | | **T2/T0** | | |
|---|---|---|---|---|
| | **PLACEBO** | **t test** | **COMPLEMENT ALIMENTAIRE** **A** | **t test** |
| | **(n=9)** | **pairé** | **(n=9)** | **pairé** |
| **VIM** | 1,02±0,36 | NS | 1,24±0,25 | 0,03 |
| **DEC** | 1,33±0,58 | NS | 1,40±0,51 | 0,03 |
| **FIBMOD** | 1,17±0,44 | NS | 1,48±0,57 | 0,02 |
| **LUM** | 1,25±0,53 | NS | 1,40±0,30 | 0,002 |
| **XYL** | 0,92±0,27 | NS | 1,25±0,27 | 0,01 |

| | | | | |
|---|---|---|---|---|
| VIM : vimentine ; DEC : décorine ; LUM : lumican ; FIBMOD : fibromoduline ; XYL: xylosyltransférase. | | | | |

Dans le groupe ayant consommé le complément alimentaire, l'ARNm codant pour la vimentine est significativement augmenté (p=0,03). Dans les cellules d'origine mésenchymateuse, telles que les fibroblastes et les cellules endothéliales, la vimentine est un composé structural majeur des filaments intermédiaires. Ce réseau du cytosquelette est directement impliqué dans les fonctions mécaniques cellulaires. En particulier, une faible expression de la vimentine affecte les processus de cicatrisation. Les cellules déficitaires en vimentine présentent notamment une faible stabilité mécanique ainsi qu'une motilité et des propriétés contractiles réduites. De plus, la vimentine participe à l'organisation spatiale des complexes focaux, à l'organisation des microfilaments actine-dépendants ainsi qu'aux interactions avec la matrice extracellulaire. Certaines de ces interactions sont directement affectées par le vieillissement cutané à l'instar de celles qui influent sur le contrôle de la migration, de la prolifération et de la contraction cellulaire ou encore sur le contrôle du phénotype métabolique.

L'étude de l'ARNm de la vimentine indique donc que le complément alimentaire A agit favorablement sur le cytosquelette des fibroblastes. C'est le signe d'une activité accrue de la cellule.

Par ailleurs, l'ARNm codant pour la décorine est surexprimé de manière significative suite à la prise du complément alimentaire A (p=0,03), alors que l'expression de cet ARNm n'est pas modifiée suite à la prise du placebo. La décorine est une petite protéine glycosylée (protéoglycan) qui appartient à la famille des Small Leucine-Rich Proteoglycans (SLRP). La décorine est présente dans l'ensemble du derme, mais absente de l'épiderme. Elle est synthétisée et sécrétée par les fibroblastes. Son rôle principal est la régulation de la fibrillogénèse du collagène. En effet, la décorine contribue à la bonne cohésion des fibres de collagène : en se fixant sur les fibres, elle leur permet de s'organiser entre elles pour former un faisceau de collagène. Il a été montré que la décorine diminue au cours du vieillissement.

Les résultats sur la décorine montrent que le complément alimentaire A semble présente un effet stabilisateur sur la structure des fibres de collagène et contribue donc à améliorer la qualité de la matrice extracellulaire du derme.

Après prise du complémentaire, on note aussi une augmentation significative des ARNm codants pour d'autres Small Leucine-Rich Proteoglycans (SLRP), en particulier ceux du lumican (p=0,002) et de la fibromodulline (p=0,02) qui sont fortement impliqués dans la fibrillogénèse et l'hydratation des espaces périfibrillaires.

Enfin, un autre ARNm a été surexprimé de manière significative suite à la prise du complément alimentaire A. Il s'agit de l'ARNm codant pour la xylosyltransférase (p=0,01) qui est la première enzyme intervenant dans la synthèse des protéoglycanes de type chondroïtine sulfate ou dermatane sulfate dans les fibroblastes. La xylosyltansférase est l'enzyme initiant la glycosylation, c'est-à-dire la fixation des sucres sur la chaîne protéique centrale du protéoglycane. Les protéoglycanes passent ensuite dans la matrice extracellulaire où ils forment un gel hydraté en absorbant les molécules d'eau. Dans ce gel baignent les cellules dermiques ainsi que les fibres de collagène et d'élastine. Les protéoglycanes jouent un rôle non seulement de réservoir hydrique permettant de résister aux forces de compression mais aussi un rôle organisationnel dans la structure de la matrice et de transmission des informations. Les protéoglycanes diminuent avec l'âge ce qui entraîne une dégradation du gel matriciel. Le complément alimentaire A en agissant sur la xylosyltransférase participe en partie à la restauration de la qualité de ce gel. En fixant mieux les molécules d'eau, ce gel matriciel hydraté permet de « regonfler » le derme et donc améliorer l'état de surface de la peau.

### Conclusion

L'ensemble de ces résultats montre que le complément alimentaire « A » a une action globale sur la qualité du derme :
1) en augmentant le métabolisme des fibroblastes *via* l'augmentation de la transcription du gène de la vimentine, protéine clé du cytosquelette ;
2) en améliorant le gel hydraté de la matrice extracellulaire dans lequel baignent les fibres et les cellules du derme, *via* l'augmentation de la transcription du gène de la xylosyltransférase, première enzyme intervenant dans la formation des protéoglycans ;
3) en structurant les fibres de collagène *via* l'augmentation de la transcription du gène du lumican, de la fibromodulline et de la décorine, des protéoglycanes intervenant dans la fibrillogénèse.

### Exemple 2 : Evaluation de l'efficacité du complément alimentaire A versus placebo sur les propriétés biomécaniques de la peau

La formule du complément alimentaire et sa posologie d'administration sont conformes à celles mises en oeuvre en exemple 1.

**Objectif de l'essai clinique :** Etude de l'influence de la prise du complément alimentaire A sur l'altération des propriétés biomécaniques de la peau et du microrelief cutané.

### Méthodologie

Etude monocentrique en double aveugle sur 2 groupes dont un placebo. Une procédure de randomisation sous contraintes a été réalisée pour assurer une meilleure comparabilité des groupes à l'inclusion.

Soixante douze volontaires sains de sexe féminin, âgés de 40 à 65 ans correspondant aux critères d'inclusion et de non-inclusion ont participé à cette étude d'une durée totale de 6 mois.
Complément alimentaire A : n=49.
Placebo : n=23 L'efficacité du complément alimentaire A a été évalué :
- par un dermatologue aux moyens de différents atlas photographiques
- par un dermatologue selon un score clinique en 6 points de 0 à 5. Plus l'état du paramètre est considéré comme bon, plus la note se rapproche de 5 et inversement.
- par des mesures instrumentales (Torquemètre® et analyse d'images à partir d'empreintes cutanées).
- par un questionnaire d'auto-évaluation renseigné par les volontaires.

### Mesure avec le Torquemètre^{®}

Le Torquemètre^{®} est un dispositif non-invasif. La tête de mesure du DTM est composée d'un disque central mobile de 20 mm de diamètre et d'une plaque circulaire fixe. Ce dispositif est apposé sur la peau par l'intermédiaire d'un ruban adhésif double face concentrique fixe. L'angle de rotation du disque central est mesuré par un capteur angulaire d'une très grande résolution. Lors de l'application de la tête de mesure, le disque central pivote. Une contrainte de torsion d'un angle Ue est alors appliquée à la zone de peau comprise entre le disque central mobile et l'anneau fixe périphérique (déformation rapide). Puis l'angle de rotation continue à croître à vitesse réduite d'un angle Uv.

Après l'arrêt du couple de torsion, la peau revient à son état initial en deux temps, rapide (déformation Ur) et lente jusqu'à l'origine.

Les zones précises de mesure sont repérées à l'aide d'un masque de forme circulaire Les paramètres mesurés sont (Ue, Uv, Ur).

### Analyse d'images à partir d'empreintes cutanées

Une réplique négative des rides de la surface cutanée est réalisée à l'aide de caoutchouc siliconé. Pour analyse, cette empreinte est éclairée par une lumière rasante qui génère des ombres portées derrière chaque ride.

L'analyse des rides est réalisée avec le logiciel Toposurf.

### Résultats

### Evaluation dermatologique au moyen des atlas photographiques

**Table 2 : Evolution des signes cutanés- Complément alimentaire A**

| PARAMETRES | T0 (moyenne ± DS) | T12 (moyenne ± DS) | T12/T0 Test de Tukey pour comparaisons multiples | T24 (moyenne ± DS) | T24/T0 Test de Tukey pour comparaisons multiples | Analyse de la variance |
|---|---|---|---|---|---|---|
| Profondeur des rides de la patte d'oie | 3.3 ± 1.1 | 2.6 ± 0.9 | AMELIORATION p=0.003 | 2.8 ± 1.0 | AMELIORATION p=0.045 | AMELIORATION p=0.004 |
| Poches | 2.4 ± 1.2 | 2.0 ± 1.4 | NS | 1.8 ± 1.2 | AMELIORATION p=0.034 | AMELIORATION p=0.042 |
| Rides de la commissure des lèvres | 2.7 ± 1.7 | 1.9 ± 1.7 | AMELIORATION p=0.048 | 1.9 ± 1.7 | *NS (tendance, p*=*0.055)* | AMELIORATION p=0.027 |

| | | | | | | |
|---|---|---|---|---|---|---|
| DS : déviation standard | | | | | | |

**Table 3 : Evolution des signes cutanés- Placebo**

| PARAMETRES | T0 (moyenne ± DS) | T12 (moyenne ± DS) | T12/T0 Test de Tukey pour comparaisons multiples | T24 (moyenne ± DS) | T24/T0 Test de Tukey comparaisons multiples | Analyse de la variance |
|---|---|---|---|---|---|---|
| Profondeur des rides de la patte d'oie | 3.3 ± 1.3 | 2.8 ± 1.2 | NS | 2.7 ± 1.1 | NS | NS |
| Poches | 1.9 ± 1.1 | 1.9 ± 1.2 | NS | 2.0 ± 1.3 | NS | NS |
| Rides de la commissure des lèvres | 2.5 ± 1.5 | 1.6 ± 1.5 | NS | 2.2 ± 1.8 | NS | NS |

| | | | | | | |
|---|---|---|---|---|---|---|
| DS : déviation standard ; NS : non statistiquement significatif | | | | | | |

Dans le groupe ayant consommé le complément alimentaire A, la profondeur des rides de la patte d'oie est statistiquement diminuée dès 12 semaines. La prise du complément alimentaire A entraîne également une diminution statistiquement significative des rides de la commissure des lèvres dès 12 semaines, ainsi que des poches sous les yeux à 24 semaines. Ces paramètres n'évoluent pas dans le groupe placebo.

### Evaluation dermatologique selon le score clinique

**Table 4 : Evolution des signes cutanés- Complément alimentaire A**

| PARAMETRES | T0 (moyenne ± DS) | T12 (moyenne ± DS) | T12/T0 Test de Tukey pour comparaisons multiples | T24 (moyenne ± DS) | T24/T0 Test de Tukey pour comparaisons multiples | Analyse de la variance |
|---|---|---|---|---|---|---|
| Souplesse de la peau | 2.7 ± 0.9 | 3.4 ± 0.8 | AMELIORATION p=3.32E-04 | 3.6 ± 0.8 | AMELIORATION p=3.36E-04 | AMELIORATION p=2.19E-06 |
| Ridules | 2.5 ± 1.0 | 2.9 ± 1.0 | NS | 3.3 ± 1.0 | AMELIORATION p=0.001 | AMELIORATION p=0.001 |
| Rides du visage | 2.3 ± 1.0 | 2.9 ± 1.0 | AMELIORATION p=0.01 | 2.8 ± 0.9 | AMELIORATION p=0.032 | AMELIORATION p=0.007 |
| Rides du décolleté | 3.2 ± 1.4 | 3.2 ± 1.0 | NS | 4.1 ± 1.1 | AMELIORATION p=0.001 | AMELIORATION p=1.83E-04 |

| | | | | | | |
|---|---|---|---|---|---|---|
| DS : déviation standard ; NS : non statistiquement significatif | | | | | | |

**Table 5 : Evolution des signes cutanés- Placebo**

| PARAMETRES | T0 (moyenne ± DS) | T12 (moyenne ± DS) | T12/T0 Test de Tukey pour comparaisons multiples | T24 (moyenne ± DS) | T24/T0 Test de Tukey pour comparaisons multiples | Analyse de la variance |
|---|---|---|---|---|---|---|
| Souplesse de la peau | 2.7 ± 0.9 | 3.2 ± 0.8 | NS | 3.2 ± 0.9 | NS | NS |
| Ridules | 2.9 ± 1.1 | 3.2 ± 1.0 | NS | 3.5 ± 0.9 | NS | NS |
| Rides du visage | 3.0 ± 1.1 | 3.2 ± 1.1 | NS | 3.3 ± 1.3 | NS | NS |
| Rides du décolleté | 4.0 ±0.7 | 3.3 ± 1.1 | DETERIORATION p=0.04₇ | 4.5 ± 1.1 | NS | p=0.001 |

| | | | | | | |
|---|---|---|---|---|---|---|
| DS : déviation standard ; NS : non statistiquement significatif | | | | | | |

Le complément alimentaire A améliore de manière statiquement significative les signes cutanés que sont les rides et ridules, ainsi que la souplesse de la peau. Le placebo ne modifie pas ces paramètres.

### Evaluation instrumentales

### Mesure avec le Torquemètre® après 6 mois de suplémentation avec le complément alimentaire A ou le placebo.

**Table 6 : Evolution des propriétés biomécaniques de la peau- Complément alimentaire A**

| PARAMETRES | T0 (moyenne ± DS) | T24 (moyenne ± DS)) | T24/T0 Analyse de la variance |
|---|---|---|---|
| Ue (extensibilité) | 2.91 ± 0.571 | 3.207 ± 0.687 | AMELIORATION p=0.023 |
| Uv (plasticité) | 1.894 ± 0.438 | 2.085 ± 0.376 | AMELIORATION p=0.024 |
| Ur (tonicité) | 1.099 ± 0.175 | 1.253 ± 0.240 | AMELIORATION p=0.001 |

| | | | |
|---|---|---|---|
| DS : déviation standard | | | |

**Table 7 : Evolution des propriétés biomécaniques de la peau- Placebo**

| PARAMETRES | T0 (moyenne ± DS) | T24 (moyenne ± DS) | T24/T0 Analyse de la variance |
|---|---|---|---|
| Ue (extensibilité) | 3.189 ± 0.695 | 3.179 ± 0.645 | NS |
| Uv (plasticité) | 1.960 ± 0.389 | 2.092 ± 0.340 | NS |
| Ur (tonicité) | 1.131 ± 0.167 | 1.209 ± 0.168 | NS |

| | | | |
|---|---|---|---|
| DS : déviation standard ; NS : non statistiquement significatif | | | |

Après 6 mois de prise du complément alimentaire A, les paramètres relatifs aux propriétés biomécaniques de la peau sont statistiquement améliorés. Ces paramètres ne sont pas modifiés par la prise du placebo.

### Analyse d'images à partir d'empreintes cutanées après 6 mois de suplémentation avec le complément alimentaire A ou le placebo.

**Table 8 : Evolution de la topographie cutanée- Complément alimentaire A**

| PARAMETRES | T0 (moyenne ± DS) | T24 (moyenne ± DS) | T24/T0 Analyse de la variance |
|---|---|---|---|
| SRvm (hauteur moyenne des vallées) | 0.059 ± 0.009 | 0.053 ± 0.01 | AMELIORATION p=0.026 |
| Densité<Z1 (densité des sillons avec une hauteur ≤ à 10 µm) | 8.06 ± 7.187 | 14.402 ± 10.192 | AMELIORATION p=0.019 |
| Densité>Z2 (densité des sillons avec une hauteur > to 20 µm) | 73.307 ± 14.935 | 55.733 ± 18.878 | AMELIORATION p=0.001 |
| Anisotropie (densité des sillons en fonction de leur hauteur et de leur orientation) | 43.926 ± 14.561 | 34.011 ± 12.114 | AMELIORATION p=0.016 |

| | | | |
|---|---|---|---|
| DS : déviation standard | | | |

**Table 9 : Evolution de la topographie cutanée- Placebo**

| PARAMETRES | T0 (moyenne ± DS) | T24 (moyenne ± DS) | T24/T0 Analyse de la variance |
|---|---|---|---|
| SRvm (hauteur moyenne des vallées) | 0.059 ± 0.019 | 0.053 ± 0.01 | NS |
| Densité<Z1 (densité des sillons avec une hauteur ≤ à 10 µm) | 13.763 ± 12.956 | 16.76 ± 8.729 | NS |
| Densité>Z2 (densité des sillons avec une hauteur > to 20 µm) | 65.231 ± 22.12 | 52.168 ± 18.494 | NS |
| Anisotropie (densité des sillons en fonction de leur hauteur et de leur orientation) | 39.117 ± 16.255 | 33.224 ± 13.099 | NS |

| | | | |
|---|---|---|---|
| DS : déviation standard ; NS : non statistiquement significatif | | | |

Le relief cutané est très fin et visible uniquement à l'aide d'une loupe dermatologique chez les enfants, alors qu'il devient visible à l'oeil nu chez les personnes âgées. Par ailleurs, le relief cutané est corrélé avec les propriétés mécaniques du derme. Les fibres de collagène du derme profond, qui s'entrecroisent, maintiennent la peau tendue chez les personnes jeunes en formant un réseau serré. Ce réseau se désorganise avec l'âge, la peau s'affaisse, et les sillons deviennent plus profonds. La topographie cutanée révèle un réseau particulier : des sillons parallèles et entrecroisés formant des rectangles, des carrés, des trapèzes, des losanges et des triangles. Les lignes primaires sont larges et profondes de 20 à 100 µm selon le site et l'âge. Les lignes secondaires sont étroites.

La prise du complément alimentaire A pendant 6 semaines entraine une amélioration statistiquement significative de l'anisotropie cutanée, une augmentation de la densité des sillons secondaires (Densité<Z1) et une diminution des sillons primaires (Densité>Z2). Ces paramètres ne sont pas modifiés dans le groupe placebo. En d'autres termes, le complément alimentaire A entraîne une réapparition des sillons secondaires (Densité<Z1) caractéristiques des peaux jeunes, une diminution de la profondeur des sillons primaires (Densité>Z2) caractéristiques des peaux âgées et une diminution de l'anisotropie cutanée qui augmente normalement avec l'âge.

### Auto-évaluation par les volontaires

La qualité des ongles a été évaluée par les volontaires au moyen d'une échelle visuelle analogique (0 cm : mauvaise qualité ; 10 cm : bonne qualité). Une augmentation de la mesure est donc synonyme d'amélioration.

**Table 10 : Evolution de la qualité des ongles-Complément alimentaire A**

| PARAMETRE | T0 (moyenne ± DS) | T6 (moyenne ± DS) | T6/T0 Test de Tukey pour comparaisons multiples | T12 (moyenne ± DS) | T12/T0 Test de Tukey pour comparaisons multiples | T24 (moyenne ± DS) | T24/T0 Test de Tukey pour comparaisons multiples |
|---|---|---|---|---|---|---|---|
| Qualité des ongles | 4.24 ± 2.96 | 6.13 ± 2.11 | AMELIORATION p=4.68E-04 | 6.48 ± 2.16 | AMELIORATION p=2.19E-05 | 6.48 ± 1.93 | AMELIORATION p=2.11E-05 |
| | | | | | | | |

| | | | | | | | |
|---|---|---|---|---|---|---|---|
| DS : déviation standard | | | | | | | |

**Table 11 : Evolution de la qualité des ongles-Placebo**

| PARAMETRE | T0 (moyenne ± DS) | T6 (moyenne ± DS) | T6/T0 Test de Tukey pour comparaisons multiples | T12 (moyenne ± DS) | T12/T0 Test de Tukey pour comparaisons multiples | T24 (moyenne ± DS) | T24/T0 Test de Tukey pour comparaisons multiples |
|---|---|---|---|---|---|---|---|
| Qualité des ongles | 4.85 ± 2.90 | 5.83 ± 2.26 | NS | 6.47 ± 2.23 | NS | 6.52 ± 2.35 | NS |

| | | | | | | | |
|---|---|---|---|---|---|---|---|
| DS : déviation standard ; NS : non statistiquement significatif | | | | | | | |

La qualité des ongles est améliorée de manière statistiquement significative chez les volontaires prenant le complément alimentaire A. La prise du placebo ne modifie pas ce paramètre.

### Conclusion

L'ensemble de ces résultats montre que le complément alimentaire A permet de corriger les signes notamment cutanés indésirables liés à une perte des performances biomécaniques en particulier de la peau en améliorant la topographie ainsi que le relief cutanés. Le complément alimentaire A aide à restructure la peau de l'intérieur pour une peau plus ferme et pour des résultats tangibles en surface.

## Revendications

1. Utilisation cosmétique par voie orale d'une association de lycopène, de vitamine C, de vitamine E et d'au moins un composé polyphénol issu d'écorce de pin à titre d'unique principe actif, destinée à prévenir et/ou traiter les signes cutanés choisis parmi : les rides ou les ridules au niveau des yeux, et les poches ou les cernes sous les yeux.

2. Utilisation selon la revendication 1, **caractérisée en ce que** la valeur du rapport entre la teneur en poids en composé polyphénol par rapport à la somme des teneurs en poids en lycopène, vitamine C et vitamine E est comprise entre 0,05 et 1,2.

3. Utilisation selon l'une quelconque des revendications précédentes, **caractérisée en ce que** la dose journalière en lycopène est comprise entre 1 et 22 mg.

4. Utilisation selon l'une quelconque des revendications précédentes, **caractérisée en ce que** la dose journalière en vitamine C est comprise entre 10 et 105 mg.

5. Utilisation selon l'une quelconque des revendications précédentes, **caractérisée en ce que** la dose journalière en vitamine E est comprise entre 1 et 17 mg.

6. Utilisation selon l'une quelconque des revendications précédentes, **caractérisée en ce que** la dose journalière en composé polyphénol est comprise entre 6 et 75 mg.

7. Utilisation selon l'une quelconque des revendications précédentes, **caractérisée en ce qu'**elle est destinée à prévenir et/ou traiter les poches péri-oculaires et **en ce que** la dose journalière en lycopène est comprise entre 2 et 11 mg, et/ou la dose journalière en vitamine C est comprise entre 20 et 70 mg, et/ou la dose journalière en vitamine E est comprise entre 3 et 12 mg et/ou la dose journalière en composé polyphénol est comprise entre 13 et 50 mg.

8. Utilisation selon l'une quelconque des revendications précédentes, **caractérisée en ce que** le composé polyphénol issu d'écorce de pin présente une teneur en trimères phénoliques pouvant aller de 5 à 25 % en poids, par rapport à son poids total.

9. Utilisation selon l'une quelconque des revendications précédentes, **caractérisée en ce que** le composé polyphénol issu d'écorce de pin présente une teneur en dimères polyphénoliques d'au moins 5 % en poids par rapport à son poids total.

10. Utilisation selon l'une quelconque des revendications précédentes, **caractérisée en ce que** le composé polyphénol issu d'écorce de pin présente de 2 à 15 % en poids, d'acides phénoliques de type acide ferrulique, acide p-coumarique, acide caféique et acide protocatéchique, par rapport à son poids total.

## Patentansprüche

1. Kosmetische orale Anwendung einer Kombination von Lycopin, Vitamin C, Vitamin E und mindestens einer Polyphenol-Verbindung aus Pinienrinde als einzigem Wirkstoff, die zur Vorbeugung und/oder Behandlung von Hautzeichen ausgelegt ist, die ausgewählt sind aus: Falten oder Fältchen um die Augen, Tränensäcken oder Ringe unter den Augen.

2. Anwendung nach Anspruch 1, **dadurch gekennzeichnet, dass** der Wert des Verhältnisses zwischen dem Gewichtsgehalt an Polyphenolverbindung bezogen auf die Summe der Gewichtsgehalte an Lycopin, Vitamin C und Vitamin E zwischen 0,05 und 1,2 beträgt.

3. Anwendung nach einem der vorangehenden Ansprüche, **dadurch gekennzeichnet, dass** die Tagesdosis an Lycopin zwischen 1 und 22 mg beträgt.

4. Anwendung nach einem der vorangehenden Ansprüche, **dadurch gekennzeichnet, dass** die Tagesdosis an Vitamin C zwischen 10 und 105 mg beträgt.

5. Anwendung nach einem der vorangehenden Ansprüche, **dadurch gekennzeichnet, dass** die Tagesdosis an Vitamin E zwischen 1 und 17 mg beträgt.

6. Anwendung nach einem der vorangehenden Ansprüche, **dadurch gekennzeichnet, dass** die Tagesdosis an Polyphenol-Verbindung zwischen 6 und 75 mg beträgt.

7. Anwendung nach einem der vorangehenden Ansprüche, **dadurch gekennzeichnet, dass** sie dazu ausgelegt ist, Tränensäcke um die Augen zu verhindern und/oder zu behandeln und dadurch, dass die Tagesdosis an Lycopin zwischen 2 und 11 mg beträgt, und/oder die Tagesdosis an Vitamin C zwischen 20 und 70 mg beträgt, und/oder die Tagesdosis an Vitamin E zwischen 3 und 12 mg beträgt und/oder die Tagesdosis an Polyphenol-Verbindung zwischen 13 und 50 mg beträgt.

8. Anwendung nach einem der vorangehenden Ansprüche, **dadurch gekennzeichnet, dass** die Polyphenol-Verbindung aus Pinienrinde einen Gehalt an Phenol-Trimeren aufweisen kann, der von 5 bis 25 Gew.-%, bezogen auf ihr Gesamtgewicht, betragen kann.

9. Anwendung nach einem der vorangehenden Ansprüche, **dadurch gekennzeichnet, dass** die Polyphenol-Verbindung aus Pinienrinde einen Gehalt an Polyphenol-Dimeren von mindestens 5 Gew.-%, bezogen auf ihr Gesamtgewicht, aufweisen kann.

10. Anwendung nach einem der vorangehenden Ansprüche, **dadurch gekennzeichnet, dass** die Polyphenol-Verbindung aus Pinienrinde 2 bis 15 Gew.-% Phenolsäuren von Typ Ferulasäure, p-Cumarinsäure, Kaffeesäure und Protokatechinsäure, bezogen auf ihr Gesamtgewicht, aufweisen kann.

## Claims

1. A cosmetic use via oral route of a combination of lycopene, of vitamin C, of vitamin E and of at least one polyphenol compound derived from pine bark as sole active ingredient, intended to prevent and/or treat cutaneous signs selected from among: lines or wrinkles at eye level, bags or dark rings under the eyes.

2. The use according to claim 1, **characterized in that** the value of the ratio between the weight content of polyphenol compound and the sum of the weight contents of lycopene, vitamin C and vitamin E is between 0.05 and 1.2.

3. The use according to any of the preceding claims, **characterized in that** the daily dose of lycopene is between 1 and 22 mg.

4. The use according to any of the preceding claims, **characterized in that** the daily dose of vitamin C is between 10 and 105 mg.

5. The use according to any of the preceding claims, **characterized in that** the daily dose of vitamin E is between 1 and 17 mg.

6. The use according to any of the preceding claims, **characterized in that** the daily dose of polyphenol compound is between 6 and 75 mg.

7. The use according to any of the preceding claims, **characterized in that** it is intended to prevent and/or treat periocular bags and **in that** the daily dose of lycopene is between 2 and 11 mg and/or the daily dose of vitamin C is between 20 and 70 mg and/or the daily dose of vitamin E is between 3 and 12 mg and/or the daily dose of polyphenol compound is between 13 and 50 mg.

8. The use according to any of the preceding claims, **characterized in that** the polyphenol compound derived from pine bark has a phenolic trimer content of possibly ranging from 5 to 25 % by weight, relative to its total weight.

9. The use according to any of the preceding claims, **characterized in that** the polyphenol compound derived from pine bark has a polyphenolic dimer content of at least 5 % by weight relative to its total weight.

10. The use according to any of the preceding claims, **characterized in that** the polyphenol compound derived from pine bark contains 2 to 15 % by weight of phenolic acids of ferulic acid, p-coumaric acid, caffeic acid and protocatechuic acid, relative to its total weight.
